(19) Europäisches Patentamt
European Patent Office
Office européen des brevets

(11) Veröffentlichungsnummer: **0 137 175**
**B1**

(12) **EUROPÄISCHE PATENTSCHRIFT**

(45) Veröffentlichungstag der Patentschrift: **07.06.89**

(21) Anmeldenummer: **84109113.5**

(22) Anmeldetag: **01.08.84**

(51) Int. Cl.⁴: **C 07 D319/20**, C 07 D319/22, C 07 D307/82, C 07 D339/08, C 07 D333/62

# (54) Verfahren zur Herstellung von benzokondensierten, tetrachlorierten, heterocyclischen Verbindungen.

(30) Priorität: **11.08.83 DE 3329126**

(43) Veröffentlichungstag der Anmeldung: **17.04.85 Patentblatt 85/16**

(45) Bekanntmachung des Hinweises auf die Patenterteilung: **07.06.89 Patentblatt 89/23**

(84) Benannte Vertragsstaaten: **BE CH DE FR GB IT LI**

(56) Entgegenhaltungen:
**EP-A- 0 046 653**
**DE-A- 2 537 891**
**DE-A- 2 844 816**
**DE-A- 2 848 531**
**DE-A- 2 916 358**
**DE-A- 3 023 328**
**US-A- 2 907 798**
**JACS, 77, 1137 (1955)**
**J. Het. Chem. 15, 893-6 (1978)**
**C A 63, 9901d**
**CA 74, 125566y**
**C A 79. 31986m**
**CA85, 105395r**

(73) Patentinhaber: **BAYER AG**
**D-5090 Leverkusen 1 Bayerwerk (DE)**

(72) Erfinder: **Marhold, Albrecht, Dr.**
**Carl-Duisberg-Strasse 329**
**D-5090 Leverkusen (DE)**
Erfinder: **Klauke, Erich, Dr.**
**Eichendorffweg 8**
**D-5068 Odenthal (DE)**

EP 0 137 175 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Das erfindungsgemäße Verfahren zur Herstellung der neuen chemischen Verbindungen der Formel (I)

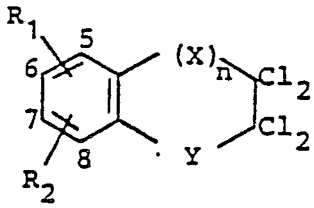

(I)

in der

$R_1$ und $R_2$ unabhängig voneinander für Wasserstoff, Fluor, Chlor, Brom, COCl, $CO_2CH_3$, Cyanid, Alkyl, Nitro, $SO_2Cl$, $SO_2F$, $OCF_3$, $SCF_3$, $CF_3$, $CCl_3$, $CBr_3$, Phenyl, substituiertes Phenyl, $OPOCl_2$, O-Alkyl, O-Aryl, S-Alkyl oder S-Aryl oder

$R_1$ und $R_2$ gemeinsam für

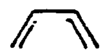

en,

X und Y unabhängig voneinander für Sauerstoff oder Schwefel stehen und

n für Null oder 1 steht,

adurch gekennzeichnet, daß man Verbindungen der Formel (II),

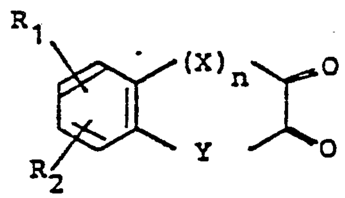

(II)

in der $R_1$, $R_2$, X, Y und n die bei Formel (I) angegebene Bedeutung haben, bei erhöhter Temperatur mit mindestens 2 Mol Phosphorpentachlorid umsetzt.

Soweit in Formel (I) $R_1$ und/oder $R_2$ für substituiertes Phenyl stehen kann es sich dabei beispielsweise um Phenylreste mit 6 bis 10 C-Atomen handeln, die durch Wasserstoff, Nitro, Fluor, Chlor, Brom, Cyanid, $C_1$- bis $C_4$-Alkyl, $SO_2Cl$, $SO_2F$, $OCF_3$, $SCF_3$, $CF_3$, $CCl_3$, $CBr_3$, O-$C_1$- bis $C_4$-Alkyl oder S-$C_1$- bis $C_4$-Alkyl substituiert sein können.

Soweit in Formel (I) $R_1$ und/oder $R_2$ für Alkyl, O-Alkyl und/oder S-Alkyl stehen kann der Alkylrest beispielsweise 1 bis 4 C-Atome enthalten.

Soweit in Formel (I) $R_1$ und/oder $R_2$ für O-Aryl und/oder S-Aryl stehen kann der Arylrest beispielsweise 6 bis 10 C-Atome enthalten.

Vorzugsweise stehen in Formel (I) $R_1$ und $R_2$ unabhängig voneinander für Wasserstoff, Fluor, Chlor, Methyl, Nitro, Phenyl oder $R_1$ und $R_2$ gemeinsam für

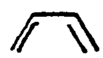

X und Y für Sauerstoff und n für Null oder 1.

Besonders bevorzugt stehen in Formel (I) $R_1$ für Wasserstoff, $R_2$ für Wasserstoff, Nitro, Methyl oder Phenyl oder $R_1$ und $R_2$ gemeinsam für

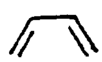

X und Y für Sauerstoff und n für 1 oder $R_1$ und $R_2$ für Wasserstoff und/oder Nitro, Y für Sauerstoff oder Schwefel und n für Null.

Die Ausgangsprodukte der Formel (II) sind zum Teil nach bekannten Verfahren (siehe z. B. Chem. Ber. 45, 157 (1912), J. Indian Chem. Soc. 18, 469 (1941), Beilstein 17, 469 und Liebigs Annalen der Chemie 1975, 1545), zum Teil auf einfache Weise durch Umsetzung von Brenzkatechin oder Brenzkatechinderivaten der Formel (III)

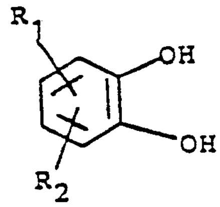

(III)

in der $R_1$ und $R_2$ die bei Formel (I) angegebene Bedeutung haben, mit Oxalylchlorid zugänglich.

Die erfindungsgemäße Umsetzung von Verbindungen der Formel (II) mit Phosphorpentachlorid zu den neuen Verbindungen der Formel (I) kann beispielsweise bei Temperaturen im Bereich von 50 bis 240 °C durchgeführt werden. Bevorzugt sind Temperaturen im Bereich von 60 bis 220 °C, besonders bevorzugt solche im Bereich von 80 bis 200 °C.

Diese Umsetzung kann im allgemeinen bei Normaldruck durchgeführt werden, sie kann jedoch auch bei erhöhtem oder erniedrigtem Druck durchgeführt werden.

Die Gegenwart von Lösungsmitteln ist bei dieser Umsetzung nicht erforderlich. In vielen Fällen ist es jedoch vorteilhaft in Gegenwart eines Lösungsmittels zu arbeiten, beispielsweise zur Verbesserung der Rührbarkeit des Reaktionsgemisches und/oder der Wärmeabfuhr. Geeignete Lösungsmittel sind beispielsweise chlorierte Kohlenwasserstoffe, insbesondere solche mit höheren Siedepunkten, wie Tetrachlorkohlenstoff, Chlorbenzol und Dichlorbenzol. Bevorzugt wird jedoch Phosphoroxychlorid als Lösungsmittel eingesetzt, da dieses auch während der Reaktion aus dem eingesetzten Phosphorpentachlorid entsteht und dann kein besonderer Aufwand für dessen Abtrennung und Rückführung entsteht.

In das erfindungsgemäße Verfahren werden mindestens 2 Mol Phosphorpentachlorid pro Mol einer Verbindung der Formel (II) eingesetzt. Überschüsse von Phosphorpentachlorid stören im allgemeinen nicht. Aus wirtschaftlichen Erwägungen arbeitet man vorzugsweise mit 2 bis 3 Mol Phosphorpentachlorid pro Mol einer Verbindung der Formel (II).

Die erfindungsgemäße Chlorierung von Verbindungen der Formel (II) ist bei Reaktionstemperaturen im Bereich von 80 bis 200 °C, im allgemeinen innerhalb von 2 bis 3 Stunden abgeschlossen. Bei tieferen (höheren) Reaktionstemperaturen können längere (kürzere) Reaktionszeiten vorteilhaft sein. Im allgemeinen wirken sich längere Reaktionszeiten, auch beim Einsatz von überschüssigem Phosphorpentachlorid, nicht negativ aus.

Im allgemeinen ist es vorteilhaft, das Phosphorpentachlorid in das erfindungsgemäße Verfahren in mehreren Teilmengen nacheinander zuzusetzen. Man kann beispielsweise so verfahren, daß man zunächst etwa 1 Mol, beispielsweise 0,8 bis 1,2 Mol Phosphorpentachlorid pro Mol der Verbindung der Formel (II) zusetzt und nach Abklingen der einsetzenden Reaktion dann das restliche Phosphorpentachlorid zufügt. Man kann ebenso das gesamte einzusetzende Phosphorpentachlorid vorlegen und die Verbindung der Formel (II) portionsweise zufügen.

Wenn man pro Mol einer Verbindung der Formel (II) etwa 1 Mol, beispielsweise 0,8 bis 1,2 Mol Phosphorpentachlorid zufügt, entstehen Verbindungen der Formel (IV)

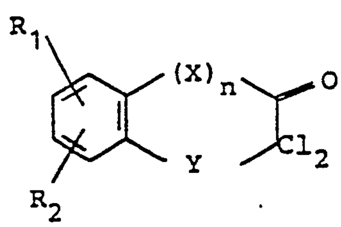

(IV)

in der $R_1$, $R_2$, X, Y und n die bei Formel (I) angegebene Bedeutung haben.

Falls erwünscht können die Verbindungen der Formel (IV) aus dem Reaktionsgemisch isoliert werden, beispielsweise durch Abtrennung des entstandenen Phosphoroxychlorids und des gegebenenfalls vorhandenen Lösungsmittels und Destillation oder Kristallisation der Verbindungen der Formel (IV). Die so isolierten Verbindungen der Formel (IV) können dann durch weitere Zugabe von Phosphorpentachlorid zu Verbindungen der Formel (I) umgesetzt werden. Es ist im Rahmen des erfindungsgemäßen Verfahrens jedoch nicht erforderlich, die Verbindungen der Formel (IV) zu isolieren. Vorzugsweise gibt man bei der Durchführung des erfindungsgemäßen Verfahrens die Gesamtmenge des Phosphorpentachlorids in zwei, vorzugsweise etwa gleichgroßen oder mehreren Teilmengen nacheinander zu und isoliert erst die Verbindungen der Formel (I) aus dem Reaktionsgemisch.

Die Aufarbeitung der Reaktionsgemische, die nach Durchführung des erfindungsgemäßen Verfahrens vorliegen, ist einfach. Das aus dem eingesetzten Phosphorpentachlorid entstandene Phosphoroxychlorid kann während und/oder nach der Umsetzung durch Destillation, gegebenenfalls zusammen mit vorhandenem Lösungsmittel, abgetrennt werden. Wenn man Reaktionstemperaturen oberhalb des

Siedepunktes von Phosphoroxychlorid erreichen will, ist die Abtrennung des Phosphoroxychlorids während der Reaktion vorzunehmen. Letzte Reste von Phosphoroxychlorid können aus dem Reaktionsgemisch vorteilhafterweise durch Anlegen eines Vakuums entfernt werden. Der nach Abtrennung des Phosphoroxychlorids und des gegebenenfalls eingesetzten Lösungsmittels verbleibende Rückstand kann dann destilliert oder kristallisiert und so die Verbindungen der Formel (I) in guten Ausbeuten und Reinheiten erhalten werden. Als Lösungsmittel für eine Reinigung durch Kristallisation kommen beispielsweise Kohlenwasserstoffe, wie Cyclohexan, Toluol und Xylol, und Alkohole, wie Methanol und Ethanol, infrage. Das abgetrennte Phosphoroxychlorid kann gegebenenfalls als Lösungsmittel für einen weiteren Ansatz verwendet werden. Gegebenenfalls im Überschuß zugefügtes und/oder unumgesetztes Phosphorpentachlorid wird zweckmäßigerweise vor der Aufarbeitung zu leichtflüchtigen Verbindungen zersetzt, beispielsweise durch Einleiten von Schwefeldioxid.

Die neuen chemischen Verbindungen der Formel (I) sind wertvolle Produkte, aus denen sich durch eine an sich bekannte Fluorierung mit Fluorwasserstoff und gegebenenfalls anschließende Nitrierung und Reduktion Aniline herstellen lassen, aus denen durch Umsetzung mit Acylisocyanaten Verbindungen zugänglich sind, die gemäß der DE-OS'en 30 23 328 und 32 23 505 eine überlegene insektizide Wirkung zeigen. Damit können diese Verbindungen mit überlegener insektizider Wirkung aus besser zugänglichen Ausgangsstoffen und auf einfachere Weise als bisher, sowie in guten Ausbeuten und Reinheiten hergestellt werden.

Es ist ausgesprochen überraschend, daß die neuen Verbindungen auf die erfindungsgemäße Weise hergestellt werden können, denn aus J.A.C.S. 77, 1137 (1955) ist bekannt, daß sich zwar Ketogruppen, nicht aber Esterfunktionen mit Phosphorpentachlorid chlorieren lassen. Beispielsweise wird dort beschrieben, daß aus der Umsetzung von Acetessigsäureethylester mit Phosphorpentachlorid β-Chlorisocrotonsäureethylester, β-Chlorcrotonsäureethylester und unumgesetzter Acetessigsäureethylester erhalten wurden, nicht jedoch 1-Methoxy-1, 1, 3, 3-tetrachlorbutan.

Die folgenden Beispiele erläutern die Herstellung von Verbindungen der Formel (I) und ihre Verwendung, ohne die vorliegende Erfindung in irgendeiner Weise zu beschränken.

## Beispiele

### Beispiele 1 bis 11

Herstellung von Ausgangsverbindungen der Formel (II)

Bei den Beispielen 1 bis 7 wurde wie folgt verfahren :

Es wurde ein Mol Brenzkatechin oder Brenzkatechinderivat (siehe Tabelle 1) in einem Gemisch aus 500 ml Toluol und 50 ml Dioxan vorgelegt und 1,05 Mol Oxalylchlorid bei 70 °C zugetropft. Danach wurde bis zum Ende der Gasentwicklung nachgerührt. Nach dem Abkühlen auf Raumtemperatur wurde das Reaktionsgemisch abfiltriert und das Festprodukt im Vakuum getrocknet. Die gleichen Ergebnisse wurden erhalten, wenn anstelle des Toluol/Dioxan-Gemisches Diethylether oder Dibutylether eingesetzt wurde. Die Ausgangsmaterialien und die Ergebnisse sind in Tabelle 1 zusammengefaßt.

(Siehe Tabelle 1 Seite 5 f.)

Tabelle 1:

| Beispiel Nr. | Ausgangsprodukt | Reaktionsprodukt | Ausbeute (d.Th.) | Schmelzpunkt (°C) |
|---|---|---|---|---|
| | 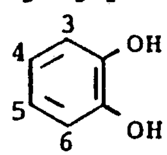 | 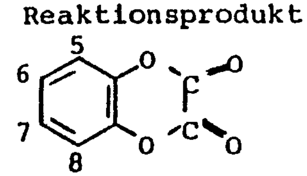 | | |
| 1 | unsubstituiert | unsubstituiert | 94,5 % | 186-188 |
| 2 | 3-Methyl | 5-Methyl | 78 % | 136-138 |
| 3 | 5-Nitro | 7-Nitro | 67 % | 180-182 |
| 4 | 5-$CO_2CH_3$ | 7-$CO_2CH_3$ | 72 % | 214-217 |
| 5 | 3-Hydroxy-5-$CO_2CH_3$ | 5-Hydroxy-7-$CO_2CH_3$ | 53 % | 130-132 |
| 6 | 4,5- 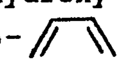 | 6,7- 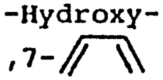 | 88 % | über 250 |
| 7 | 4-Phenyl | 6-Phenyl | 84 % | 160-162 |
| 7a | 3-OH | 5-OH | 71 % | 138-141 |

Weitere Ausgangsverbindungen der Formel (II) wurden nach Literaturangaben hergestellt (siehe Tabelle 2).

Tabelle 2

| Beispiel Nr. | Produkt | Literaturstelle |
|---|---|---|
| 8 | 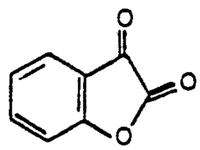 | Chem. Ber. 45, 157 (1912) |
| 9 | 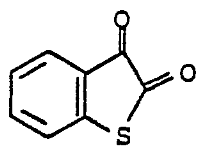 | J. Indian Chem. Soc. 18, 469 (1941), Beilstein 17, 467 |
| 10 | 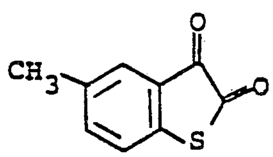 | " " |
| 11 | 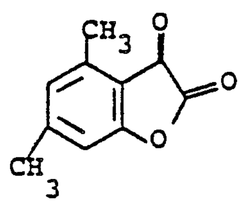 | Liebigs Annalen der Chemie 1975, 1545 |

Beispiele 12 bis 15

Herstellung von Verbindungen der Formel (IV)

1 Mol einer Dicarbonylverbindung wurde mit 1,05 Mol Phosphorpentachlorid vermischt und langsam erwärmt bis zur Verflüssigung der Reaktionsmischung. Dann wurde die Temperatur langsam gesteigert und gebildetes Phosphoroxychlorid abdestilliert. Zur Vervollständigung der Reaktion wurde die Abnahme des Phosphoroxychlorids langsam mittels eines Rückflußteilers vorgenommen und die Innentemperatur der Reaktionsmischung bis auf 200 °C gebracht. Restliches Phosphoroxychlorid wurde durch Anlegen eines Vakuums entfernt. Die Reaktionsprodukte wurden durch Destillation (dann ist der Siedepunkt angegeben) oder durch Kristallisation aus Cyclohexan/Toluol (dann ist der Schmelzpunkt angegeben) gereinigt. Die Ausgangsmaterialien und die Ergebnisse sind in Tabelle 3 zusammengefaßt.

(Siehe Tabelle 3 Seite 7 f.)

Tabelle 3

| Beispiel Nr. | Ausgangsprodukt 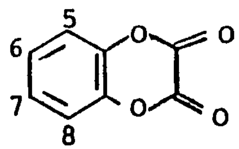 | Reaktionsprodukt 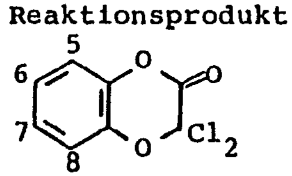 | Ausbeute (d.Th.) | Siede- oder Schmelzpunkt (°C) |
|---|---|---|---|---|
| 12 | unsubstituiert | unsubstituiert | 96 % | 130-132/ 16 mbar (Sdp.) |
| 13 | 6,7- 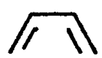 | 6,7- 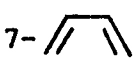 | 87 % | 170-172 (Schmp.) |
| 14 | 7-Methyl | 7-Methyl | 67 % | 142-145/ 18 mbar (Sdp.) |
| 15 | 6-Phenyl | 6-Phenyl | 89 % | 180-184/ 0,6 mbar (Sdp.) |

Beispiele 16 bis 21

Herstellung von Verbindungen der Formel (I)

1 Mol der jeweils angegebenen Ausgangsverbindung der Formel (II) wurde mit einem Mol Phosphorpentachlorid vermischt und erhitzt, bis die Mischung flüssig geworden war. Dann wurden weitere 1,2 Mol Phosphorpentachlorid zugefügt und begonnen das entstandene Phosphoroxychlorid abzudestillieren. Dabei wurde die Temperatur nach und nach bis auf 190 bis 200 °C gesteigert, um Reste an Phosphoroxychlorid überzudestillieren. Der Rückstand wurde entweder destilliert (dann ist der Siedepunkt angegeben) oder aus Methanol kristallisiert (dann ist der Schmelzpunkt angegeben). Die Ausgangsmaterialien und die Ergebnisse sind in Tabelle 4 zusammengefaßt.

Tabelle 4

| Beispiel Nr. | Ausgangsprodukt 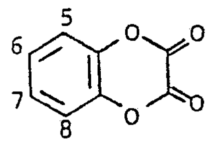 | Reaktionsprodukt 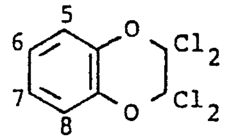 | Ausbeute (d.Th.) | Siede- und Schmelzpunkt (°C) |
|---|---|---|---|---|
| 16 | unsubstituiert | unsubstituiert | 92 % | 140-142/20 mbar (Sdp.) |
| 17 | 7-Nitro | 7-Nitro | 81 % | 81-82 (Schmp.) |
| 18 | 8-Methyl | 8-Methyl | 72 % | 158-162/20 mbar (Sdp.) |
| 19 | 6-Phenyl | 6-Phenyl | 84 % | 115-117 (Schmp.) |
| 20 | 6,7- 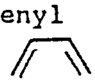 | 6,7- 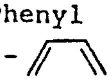 | 88 % | 175-178 (Schmp.) |
| 21 | 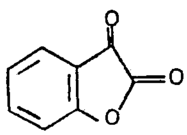 | 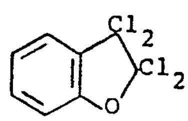 | 47 % | 54-56 (Schmp.) |

Beispiel 22

Verwendung von Verbindungen der Formel (I)

In einer Fluorierungsapparatur aus VA-Stahl wurden 150 ml Fluorwasserstoff vorgelegt und bei 0 °C 50 g 8-Nitro-2.2.3.3-tetrachlor-1.4-benzodioxen zugegeben. Anschließend wurden 3 bar Stickstoff aufgedrückt, für 3 Stunden auf 60 °C und für weitere 2 Stunden auf 100°C erhitzt, danach abgekühlt und entspannt, 1 ml Antimonpentachlorid zugefügt, erneut 3 bar Stickstoff aufgedrückt und für weitere 3 Stunden auf 120 °C erhitzt. Nach dem Abkühlen wurde der Ansatz auf Eis ausgetragen, die organische Phase mit Dichlormethan aufgenommen, mit Wasser gewaschen, getrocknet und destilliert. Es wurden 26 g Nitro-2.2.3-trifluor-3-chlor-1.4-benzodioxen mit einem Siedepunkt von 125 bis 130 °C bei 20 mbar erhalten. Diese Verbindung wurde wie in der DE-OS 30 23 328 beschrieben zu den dort beschriebenen Acylharnstoffen mit hervorragender insektizider Wirkung umgesetzt.

## Patentansprüche

1. Verfahren zur Herstellung von Verbindungen der Formel

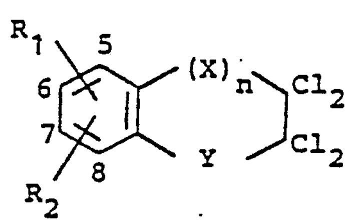

in der

$R_1$ und $R_2$ unabhängig voneinander für Wasserstoff, Fluor, Chlor, Brom, COCl, $CO_2CH_3$, Cyanid, Alkyl, Nitro, $SO_2Cl$, $SO_2F$, $OCF_3$, $SCF_3$, $CF_3$, $CCl_3$, $CBr_3$, Phenyl, substituiertes Phenyl, $OPOCl_2$, O-Alkyl, O-Aryl, S-Alkyl oder S-Aryl oder

$R_1$ und $R_2$ gemeinsam für

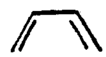

stehen,

X und Y unabhängig voneinander für Sauerstoff oder Schwefel stehen und

n für Null oder 1 steht,

dadurch gekennzeichnet, daß man Verbindungen der Formel

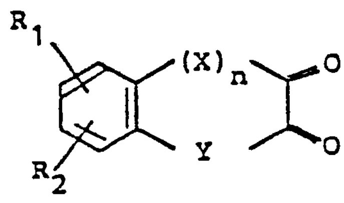

in der $R_1$, $R_2$, X, Y und n die oben angegebene Bedeutung haben, bei erhöhter Temperatur mit mindestens 2 Mol Phosphorpentachlorid umsetzt.

2. Verfahren nach Anspruch 1, dadurch gekennzeichnet, daß man die Umsetzung bei Temperaturen im Bereich von 50 bis 240 °C durchführt.

3. Verfahren nach Anspruch 2, dadurch gekennzeichnet, daß man die Umsetzung bei Temperaturen im Bereich von 80 bis 200 °C durchführt.

4. Verfahren nach Ansprüchen 1 bis 3, dadurch gekennzeichnet, daß man pro Mol der Verbindung der Formel

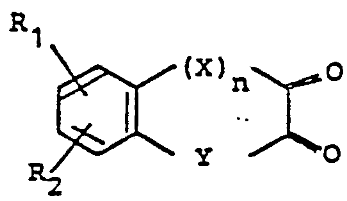

2 bis 3 Mol Phosphorpentachlorid einsetzt.

5. Verfahren nach Ansprüchen 1 bis 4, dadurch gekennzeichnet, daß man das Phosphorpentachlorid in mehreren Teilmengen nacheinander zusetzt.

6. Verfahren nach Ansprüchen 1 bis 5, dadurch gekennzeichnet, daß man pro Mol der Verbindung der Formel

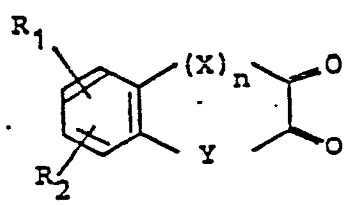

zunächst 0,8 bis 1,2 Mol Phosphorpentachlorid zusetzt, so Verbindungen der Formel

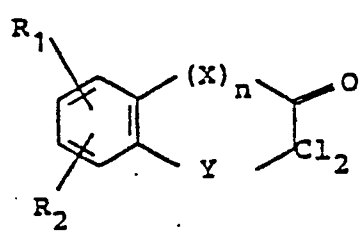

in der $R_1$, $R_2$, X, Y und n die in Anspruch 4 angegebene Bedeutung haben, herstellt und daraus durch weitere Zugabe von Phosphorpentachlorid die Verbindungen der Formel

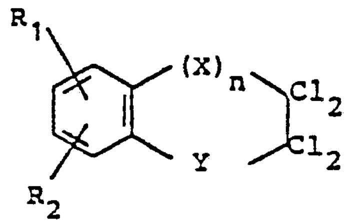

herstellt.

7. Verfahren nach Ansprüchen 1 bis 6, dadurch gekennzeichnet, daß man die Umsetzung in Gegenwart von Phosphoroxychlorid als Lösungsmittel durchführt.

## Claims

1. Process for the preparation of compounds of the formula

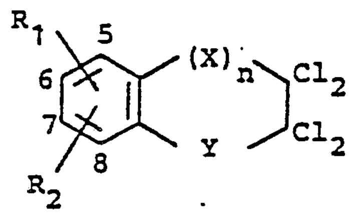

in which

$R_1$ and $R_2$ independently ot one another represent hydrogen, fluorine, chlorine, bromine, COCl, $CO_2CH_3$, cyanide, alkyl, nitro, $SO_2Cl$, $SO_2F$, $OCF_3$, $SCF_3$, $CF_3$, $CCl_3$, $CBr_3$, phenyl, substituted phenyl, $OPOCl_2$, O-alkyl, O-aryl, S-alkyl or S-aryl or

$R_1$ and $R_2$ together represent

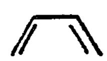

X and Y independently of one another represent oxygen or sulphur and

n represents zero or 1,

characterised in that compounds of the formula

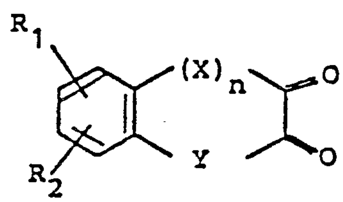

in which $R_1$, $R_2$, X, Y and n have the abovementioned meaning, are reacted with at least 2 moles of phosphorus pentachloride at elevated temperature.

2. Process according to Claim 1 characterised in that the reaction is carried out at temperatures in the range from 50 to 240 °C.

3. Process according to Claim 2, characterised in that the reaction is carried out at temperatures in the range from 80 to 200 °C.

4. Process according to Claims 1 to 3, characterised in that 2 to 3 moles of phosphorus pentachloride are employed per mole of the compound of the formula

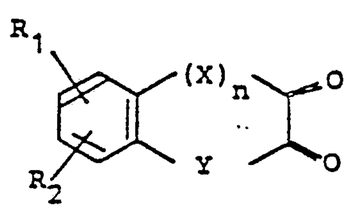

5. Process according to Claims 1 to 4, characterised in that the phosphorus pentachloride is added successively in several part amounts.

6. Process according to Claims 1 to 5, characterised in that 0.8 to 1.2 moles of phosphorus pentachloride are first added per mole of the compound of the formula

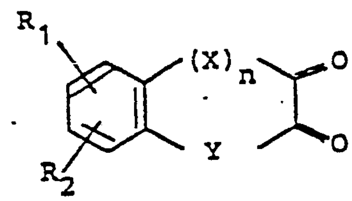

and compounds of the formula

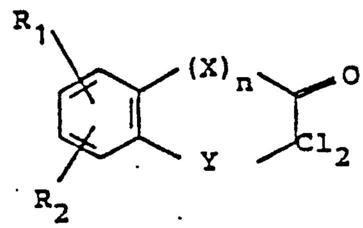

in which $R_1$, $R_2$, X, Y and n have the meaning given in Claim 4, are thus prepared, and the compounds of the formula

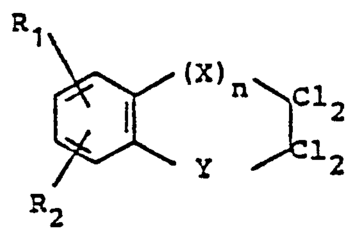

are prepared therefrom by further addition of phosphorus pentachloride.

7. Process according to Claims 1 to 6, characterised in that the reaction is carried out in the presence of phosphorus oxychloride as the solvent.

## Revendications

1. Procédé de production de composés de formule

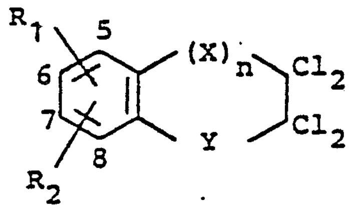

dans laquelle

$R_1$ et $R_2$ représentent, indépendamment l'un de l'autre, l'hydrogène, le fluor, le chlore, le brome, un groupe COCl, $CO_2CH_3$, cyanure, alkyle, nitro, $SO_2Cl$, $SO_2F$, $OCF_3$, $SCF_3$, $CF_3$, $CCl_3$, $CBr_3$, phényle, phényle substitué, $OPOCl_2$, O-alkyle, O-aryle, S-alkyle ou S-aryle ou bien

$R_1$ et $R_2$ forment conjointement un groupe,

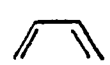

X et Y représentent, indépendamment l'un de l'autre, l'oxygène ou le soufre et

n a la valeur 0 ou 1,

caractérisé en ce qu'on fait réagir des composés de formule

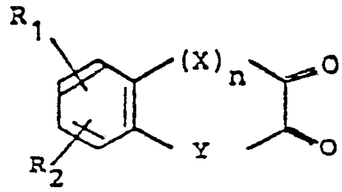

dans laquelle

$R_1$, $R_2$, X, Y et n ont la définition indiquée ci-dessus, à température élevée avec au moins 2 moles de pentachlorure de phosphore.

2. Procédé suivant la revendication 1, caractérisé en ce qu'on conduit la réaction à des températures comprises dans l'intervalle de 50 à 240 °C.

3. Procédé suivant la revendication 2, caractérisé en ce qu'on conduit la réaction à des températures comprises dans l'intervalle de 80 à 200 °C.

4. Procédé suivant les revendications 1 à 3, caractérisé en ce qu'on utilise par mole de composé de formule

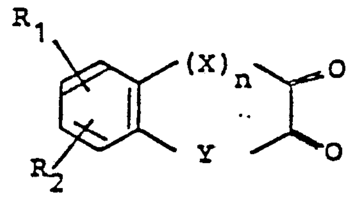

2 à 3 moles de pentachlorure de phosphore.

5. Procédé suivant les revendications 1 à 4, caractérisé en ce qu'on ajoute le pentachlorure de phosphore successivement en plusieurs portions.

6. Procédé suivant les revendications 1 à 5, caractérisé en ce qu'on ajoute par mole de composé de formule

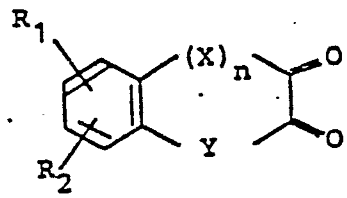

tout d'abord 0,8 à 1,2 mole de pentachlorure de phosphore, en sorte que l'on produit des composés de formule

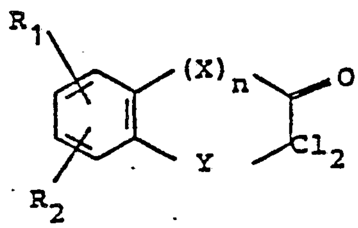

dans laquelle $R_1$, $R_2$, X, Y et n ont la définition indiquée dans la revendication 4,
et on prépare à partir de ces composés, par une nouvelle addition de pentachlorure de phosphore, les composés de formule

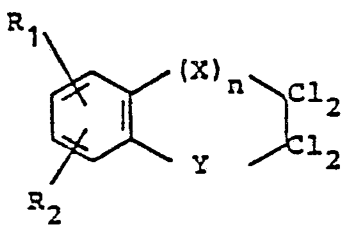

7. Procédé suivant les revendications 1 à 6, caractérisé en ce qu'on conduit la réaction en présence d'oxychlorure de phosphore comme solvant.